# EUROPEAN PATENT APPLICATION

(11) **EP 4 471 036 A1**
(43) Date of publication of application: **04.12.2024**
(21) Application number: 23745962.3
(22) Date of filing: 10.01.2023
(51) Int. Cl.: C07D 498/22

(54) **CRYSTAL FORM OF COMPOUND AND PREPARATION METHOD THEREFOR**

(30) Priority: 29.01.2022 CN 202210111070
(71) Applicant: Nanjing Innocare Pharma Tech Co., Ltd., Nanjing, Jiangsu 211100 (CN)
(72) Inventor: KONG, Norman Xianglong, Nanjing, Jiangsu 211100 (CN); FENG, Jing, Nanjing, Jiangsu 211100 (CN)
(74) Representative: Elzaburu S.L.P.
(86) International application number: PCT/CN2023/071722
(87) International publication number: WO 2023/143079

(57) **Abstract**

The present invention relates to a crystalline form of the compound (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one of formula (I) and a preparation method therefor. The crystalline form provided in the present invention has favorable properties in terms of stability and hygroscopicity, and is suitable for drug research and industrial production. The crystalline form has significant value for the optimization and development of the drug in the future.

## Description

### TECHNICAL FIELD

The present invention relates to a TRK inhibitor (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one in crystalline form and preparation method therefor.

### BACKGROUND

The heterocyclic compound (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one (referred to as the compound of formula (I) hereinafter), which is used as a TRK inhibitor, has the structure shown in formula (I) below. The preparation of the compound of formula (I) and the use thereof for treating or preventing TRK-mediated related diseases (e.g., tumors) are disclosed in the Chinese patent application CN110627812.

### SUMMARY OF THE INVENTION

In search of forms that are not particularly sensitive to the conditions under which they are tested, stored and used so that more suitable for use in the preparation of pharmaceutical formulations, with unremitting efforts, the applicant has found the crystalline form of the compound of formula (I) which are superior in terms of stability, particularly in terms of thermal stability and/or hygroscopicity.

Specifically, the present invention provides a crystalline form A of the compound (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one of formula (I) and preparation method therefor.

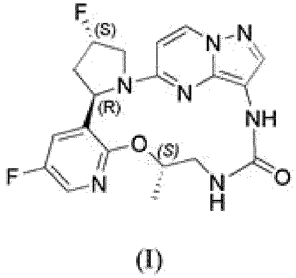

The new crystalline form A of the compound of formula (I) provided in the present invention has at least one favorable property in terms of stability and hygroscopic, which is suitable for drug research and industrial production thereby.

In one aspect, the present invention provides a crystalline form of (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one (the compound of formula (I)) which is crystalline form A, wherein an X-ray powder diffraction pattern obtained from a CuKα source has characteristic peaks at 2θ values of 19.9°±0.2°, 20.9°±0.2°, 22.2°±0.2°.

In one aspect, the present invention provides the aforementioned crystalline form A of the compound of formula (I), wherein the X-ray powder diffraction pattern thereof further has characteristic peaks at one or more of the 2θ values of 21.4°±0.2°, 9.8°±0.2°, 12.1°±0.2°.

In one aspect, the present invention provides the aforementioned crystalline form A of the compound of formula (I), wherein the X-ray powder diffraction pattern thereof further has characteristic peaks at one or more of the 2θ values of 18.0°±0.2°, 24.6°±0.2°, 11.2°±0.2°.

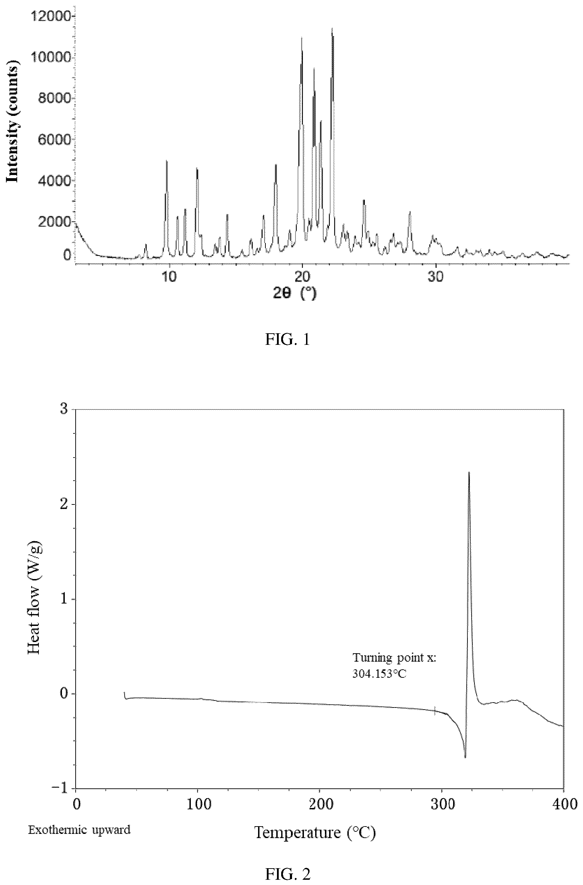

In one aspect, the present invention provides the aforementioned crystalline form A of the compound of formula (I), wherein the X-ray powder diffraction pattern substantially as shown in FIG. 1.

In one aspect, the present invention relates to a method for preparing the crystalline form A of the compound of formula (I), comprising the following steps:
dissolving the free base form of (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one in a solvent in which the free base has a solubility greater than 2 mg/mL at a temperature of between room temperature and 50°C to obtain a clear solution, followed by cooling and standing to obtain the crystalline form A, or by evaporation to precipitate crystals to obtain the crystalline form A. Wherein the solvent is selected from methanol, acetone, dichloromethane, dimethyl sulfoxide, etc., or any mixture thereof.

In one aspect, the present invention relates to a method for preparing the crystalline form A of the compound of formula (I), comprising the following steps:
the free base form of (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one was dissolved in a solvent to obtain a clear solution, then an anti-solvent was added to crystallize; wherein the solvent is a solvent in which the solubility of
(2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one is greater than 2 mg/mL; the anti-solvent is a solvent in which the solubility of
(2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one is less than 2 mg/mL. Wherein the solvent is selected from methanol, acetone, dichloromethane, dimethyl sulfoxide, etc.,
or any mixture thereof; the anti-solvent is selected from isopropyl alcohol, n-heptane, water, or any mixture thereof.

In the method for preparing the crystalline form A of the compound of formula (I) described in the present invention, the ratio of the solvent to (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one is 0.2-10 ml: 25 mg, preferably 0.8 ml: 25 mg.

In one aspect, the crystalline form A of the compound of formula (I) provided in the present invention is an anhydride.

In one aspect, the crystalline form A of the compound of formula (I) provided in the present invention, wherein when differential scanning calorimetry is performed, an endothermic peak appears when heated to 304°C (onset temperature), and the differential scanning calorimetry pattern thereof substantially as shown in FIG. 2.

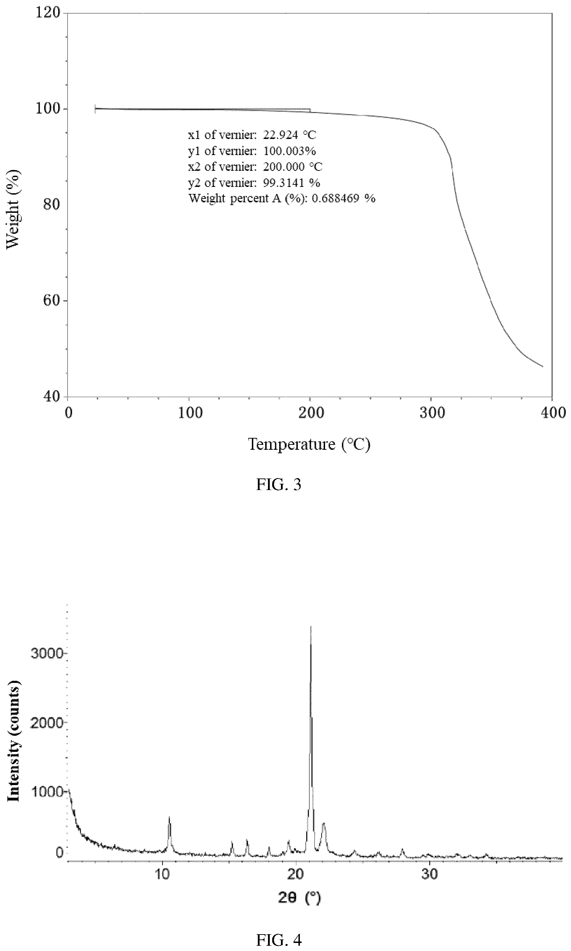

In one aspect, the crystalline form A of the compound of formula (I) provided in the present invention, wherein when thermogravimetric analysis is performed, it has a weight loss gradient of about 0.69% when heated to 200°C, and the thermogravimetric analysis pattern thereof substantially as shown in FIG. 3.

Further, the free base form of (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one is suspended and stirred in the solvent to obtain the crystalline form of the TRK inhibitor (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1 ,2)-pyrrolidincyclononan-8-one.

More further, the solvent is a solvent or mixed solvents with a solubility of 20 mg/mL>S>0.1 mg/mL.

More further, in the aforementioned preparation methods, the ratio of the solvent to the TRK inhibitor (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one is 0.2 ∼ 10 ml: 25 mg, preferably 0.8 ml: 25 mg.

More further, the temperature at which the method is performed is -20 -100 °C.

The crystalline form A of the compound of formula (I) in the present invention shows a stable state when placed under the conditions of the temperature as high as 60°C (for 1 month), humidity as high as 25°C/90%±5% RH (for 1 month) and light (for 13 days), and still shows a stable state when placed under the condition of 30°C/65% RH for 12 months. The crystalline form A of the compound of formula (I) in the present invention gains 0.46% of weight after balancing at 80% RH, indicating that it is only slightly hygroscopic; and the crystalline form A remains unchanged under different humidity conditions. In addition, as shown in the study of the suspension competition experiments in solution, the crystalline form B of the compound of formula (I) in the present invention can be transformed into the crystalline form A at room temperature, indicating that the crystalline form A of the compound of formula (I) has better thermodynamic stability.

### BRIEF DESCRIPTION OF THE DRAWING

FIG. 1 shows the XRPD pattern of the crystalline form A;
FIG. 2 shows the DSC diagram of the crystalline form A;
FIG. 3 shows the TGA diagram of the crystalline form A;
FIG. 4 shows the XRPD pattern of the crystalline form B;
FIG. 5 shows the DVS diagram of the crystalline form A;
FIG. 6 shows the XRPD comparison of the crystalline form A before and after the DVS test;
FIG. 7 shows the XRPD pattern of the suspension competition between the crystalline forms.

### DETAILED DESCRIPTION OF THE EMBODIMENTS

The invention will be further illustrated by specific examples below, but is not intended to limit the scope of protection of the present invention. The person skilled in the art may make improvements to the preparation method and the instruments used within the scope of the claims, and these improvements shall also be considered as the scope of protection of the present invention. Therefore, the attached claims shall prevail in the scope of protection of the present invention patent.

The compound (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one of formula (I) in the present invention can be prepared by methods known in the prior art. The solvents and reagents used for crystallization can be obtained by conventional ways, such as commercial sale.

Unless otherwise indicated, all terms used herein have the meanings understood by those skilled in the art or commonly used in the art.

The "stability" described in the present invention refers to that neither degradation of the crystalline form has been found nor transformation to other crystalline form has been detected in the determination of the crystalline form by analytical means such as LC detection, XRPD, etc.

The "hygroscopicity" described in the present invention refers to the characteristic of the substance's ability or degree of absorbing water under the conditions of certain temperature and humidity. Regarding to the characteristic description of hygroscopicity and the definition of hygroscopic weight gain (performing experiments according to the method of guideline 9103 of Pharmacopoeia of the People's Republic of China (2015) Volume IV, experimental conditions: 25°C±1°C, 80%±2% of relative humidity):
deliquescence: sufficient water absorption to form a solution;
very hygroscopic: hygroscopic weight gain of not less than 15%;
hygroscopic: hygroscopic weight gain of less than 15% but not less than 2%;
slightly hygroscopic: hygroscopic weight gain of less than 2% but not less than 0.2%; not or almost not hygroscopic: hygroscopic weight gain of less than 0.2%.

The abbreviations used in the present invention are explained as follows:
XRPD: X-ray powder diffraction;
DSC: differential scanning calorimetry;
TGA: thermogravimetric analysis;
DVS: dynamic vapor sorption.

The X-ray powder diffraction patterns described in the present invention are acquired on the Bruker D8 ADVANCE ray powder diffractometer. The parameters of the X-ray powder diffraction method described in the present invention are as follows:
X-ray reflection parameters: Cu, Kα;
Kα1(Å): 1.540598; Kα2(Å): 1.544426;
Kα2/Kα1 intensity ratio: 0.50;
voltage: 40 kilovolts (kV);
current: 40 milliamperes (mA);
scanning range: from 3.0 degree to 40.0 degree.

The differential scanning calorimetry (DSC) diagram described in the present invention was acquired using a TA DSC250 instrument. The method parameters for the differential scanning calorimetry (DSC) described in the present invention are as follows:
scanning rate: 10°C/min;
protective gas: nitrogen.

The thermogravimetric analysis (TGA) diagram described in the present invention was acquired using a TA TGA550 instrument. The method parameters for the thermogravimetric analysis (TGA) described in the present invention are as follows:
scanning rate: 10°C/min;
protective gas: nitrogen.

The dynamic vapor sorption (DVS) diagram described in the present invention was acquired using an Intrinsic dynamic vapor sorption instrument produced by SMS (Surface Measurement Systems Ltd.). The method parameters of the dynamic vapor sorption described in the present invention are as follows:
temperature: 25 °C;
carrier gas, flow rate: N₂, 200 mL / min;
mass variation per unit time: 0.002% / min;
relative humidity range: 0%RH - 95%RH.

The compound (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one of formula (I) used in the following examples is obtained according to the preparation method described in the invention patent application WO 2020/001415.

### Examples

### Example 1

Preparation of the crystalline form A of (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one:
25.1 mg of the compound of formula (I) in the form of free base was weighed and stirred in 0.8 mL methanol at 50°C for 2 h. The saturated solution was obtained by filtration and cooled down to 5°C at 0. 1°C/min to precipitate out as a solid, which is the crystalline form A of the compound of formula (I).

The X-ray powder diffraction data of the obtained crystalline form A is shown in Table 1; the XRPD pattern is shown in FIG. 1; the DSC diagram is shown in FIG. 2; the TGA diagram is shown in FIG. 3.

**Table 1**

| No. | 2θ angle (°) | Interplana r spacing (Å) | Relative intensity (%) | No. | 2θ angle (°) | Interplanar spacing (Å) | Relative intensity (%) |
|---|---|---|---|---|---|---|---|
| 1 | 7.73 | 11.43 | 1.4 | 30 | 24.62 | 3.61 | 23.6 |
| 2 | 8.25 | 10.71 | 6.0 | 31 | 24.94 | 3.57 | 9.8 |
| 3 | 8.83 | 10.00 | 0.5 | 32 | 25.27 | 3.52 | 4.8 |
| 4 | 9.81 | 9.01 | 43.2 | 33 | 25.56 | 3.48 | 8.5 |
| 5 | 10.64 | 8.31 | 17.8 | 34 | 26.18 | 3.40 | 3.3 |
| 6 | 11.20 | 7.89 | 21.5 | 35 | 26.63 | 3.34 | 6.4 |
| 7 | 12.09 | 7.32 | 39.7 | 36 | 26.83 | 3.32 | 8.6 |
| 8 | 12.40 | 7.13 | 9.5 | 37 | 27.13 | 3.28 | 4.9 |
| 9 | 12.74 | 6.94 | 0.9 | 38 | 27.37 | 3.26 | 5.3 |
| 10 | 13.46 | 6.57 | 5.6 | 39 | 28.05 | 3.18 | 18.5 |
| 11 | 13.80 | 6.41 | 8.5 | 40 | 28.77 | 3.10 | 0.7 |
| 12 | 14.37 | 6.16 | 19.1 | 41 | 29.80 | 3.00 | 8.7 |
| 13 | 15.46 | 5.73 | 3.0 | 42 | 30.06 | 2.97 | 6.9 |
| 14 | 16.13 | 5.49 | 7.4 | 43 | 30.27 | 2.95 | 5.0 |
| 15 | 16.64 | 5.32 | 2.1 | 44 | 31.60 | 2.83 | 3.5 |
| 16 | 17.08 | 5.19 | 17.1 | 45 | 32.32 | 2.77 | 2.7 |
| 17 | 18.01 | 4.92 | 39.3 | 46 | 32.63 | 2.74 | 1.8 |
| 18 | 18.70 | 4.74 | 2.3 | 47 | 33.07 | 2.71 | 2.7 |
| 19 | 19.05 | 4.67 | 9.0 | 48 | 33.31 | 2.69 | 2.5 |
| 20 | 19.94 | 4.45 | 96.3 | 49 | 34.04 | 2.63 | 2.9 |
| 21 | 20.50 | 4.33 | 13.8 | 50 | 34.46 | 2.60 | 2.0 |
| 22 | 20.87 | 4.25 | 82.0 | 51 | 35.05 | 2.56 | 2.5 |
| 23 | 21.37 | 4.15 | 58.0 | 52 | 35.72 | 2.51 | 1.0 |
| 24 | 21.89 | 4.06 | 10.9 | 53 | 36.21 | 2.48 | 1.0 |
| 25 | 22.22 | 4.00 | 100.0 | 54 | 36.50 | 2.46 | 2.4 |
| 26 | 23.06 | 3.85 | 12.1 | 55 | 37.26 | 2.41 | 1.3 |
| 27 | 23.38 | 3.80 | 8.8 | 56 | 37.55 | 2.39 | 2.4 |
| 28 | 23.94 | 3.71 | 6.9 | 57 | 38.73 | 2.32 | 1.8 |
| 29 | 24.20 | 3.68 | 3.9 | 58 | 39.18 | 2.30 | 1.0 |

### Example 2

Preparation of the crystalline form B of the compound of formula (I):
24.9 mg of the compound of formula (I) in the form of free base was weighed and stirred in 0.8 mL of 1,4-dioxane/acetone (1:1) at 50 °C for 2 h. The saturated solution was obtained by filtration, and transferred to the room temperature environment after cooling down to 5 °C at 0.1°C/min. A solid was obtained by evaporation in the air, which is the crystalline form B of the compound of formula (I).

The X-ray powder diffraction data of the obtained crystalline form B are shown in Table 2; the XRPD pattern is shown in FIG. 4.

**Table 2**

| No. | 2θ angle (°) | Interplan ar spacing (Å) | Relative intensity (%) | No. | 2θ angle (°) | Interplana r spacing (Å) | Relative intensit y (%) |
|---|---|---|---|---|---|---|---|
| 1 | 9.82 | 9.01 | 1.1 | 11 | 22.08 | 4.02 | 14.1 |
| 2 | 10.53 | 8.39 | 15.7 | 12 | 24.37 | 3.65 | 2.4 |
| 3 | 13.28 | 6.66 | 0.8 | 13 | 26.14 | 3.41 | 1.7 |
| 4 | 15.23 | 5.81 | 6.7 | 14 | 27.96 | 3.19 | 3.0 |
| 5 | 16.35 | 5.42 | 7.5 | 15 | 29.53 | 3.02 | 0.8 |
| 6 | 18.01 | 4.92 | 3.9 | 16 | 29.95 | 2.98 | 1.6 |
| 7 | 19.10 | 4.64 | 0.9 | 17 | 32.07 | 2.79 | 1.6 |
| 8 | 19.46 | 4.56 | 6.4 | 18 | 32.98 | 2.71 | 1.1 |
| 9 | 19.92 | 4.45 | 2.3 | 19 | 34.25 | 2.62 | 2.0 |
| 10 | 21.12 | 4.20 | 100.0 | 20 | 36.63 | 2.45 | 0.8 |

### Example 3

Stability assessment of the crystalline form A:
The solid sample of crystalline form A was exposed to high temperature, high humidity and light (open) for an one month accelerated stability study to observe the purity of the sample and the stability of the crystalline form.

The solid sample of crystalline form A was placed under the 30°C/65%RH (closed) for a 12 months stability study to observe the purity of the sample and the stability of the crystalline form (XRPD pattern).

The results are shown in Table 3.

**Table 3**

| Experimental condition | Purity (%) | Crystalline form |
|---|---|---|
| 0 month | 98.9 | - |
| Acceleration for 1 month (60°C) | 98.3 | Unchanged |
| Acceleration for 1 month (25°C, 90% ± 5%RH) | 98.5 | Unchanged |
| Acceleration for 13 days (10 days of light exposure, 3 days of UV exposure) | 99.4 | Unchanged |
| 12 months | 99.4 | Unchanged |

It can be seen from the test results shown in Table 3 that the crystalline form of the sample remains unchanged under both accelerated and 12 months conditions, and the purity changes little. The crystalline form A can maintain stable under the above conditions, indicating that it has good stability.

### Example 4

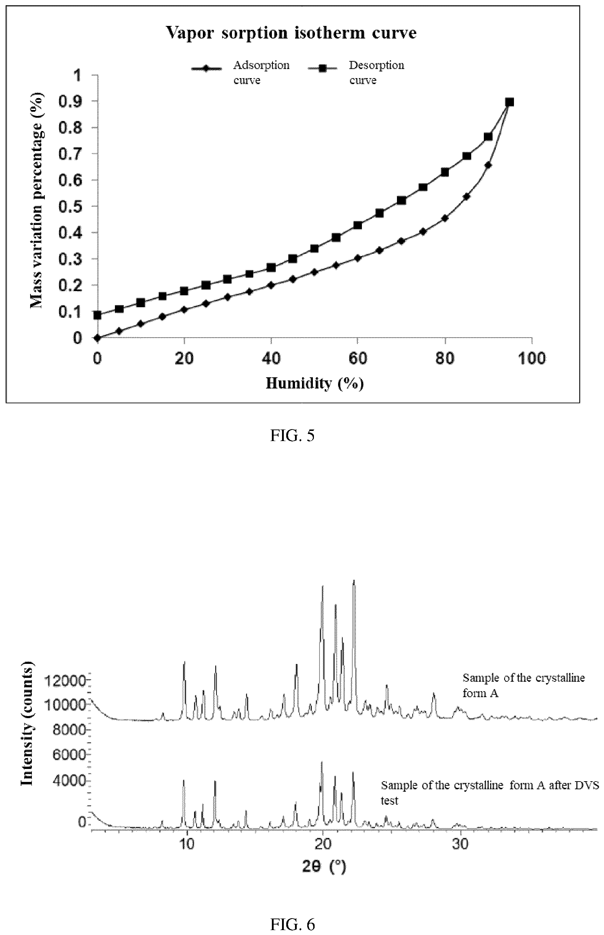

Hygroscopicity assessment of the crystalline form A:
About 30 mg of the crystalline form A of the present invention was taken and subjected to a dynamic vapor sorption test using a dynamic vapor sorption (DVS). Specifically, the dynamic vapor sorption (DVS) diagrams were acquired on an Intrinsic dynamic vapor sorption produced by SMS (Surface Measurement Systems Ltd.). The parameters of the dynamic vapor sorption were as follows: temperature: 25 °C; carrier gas and flow rate: N₂, 200 mL/min; mass variation per unit time: 0.002%/min; relative humidity range: 0%RH to 95%RH. The XRPD was measured before and after the test. The results show that the crystalline form A of the present invention has a weight gain of 0.46% at 80% relative humidity, indicating slight hygroscopic. The DVS diagram of crystalline form A is shown in FIG. 5. The crystalline form remained unchanged before and after the dynamic vapor sorption test, and the XRPD comparison thereof is shown in FIG. 6.

### Example 5

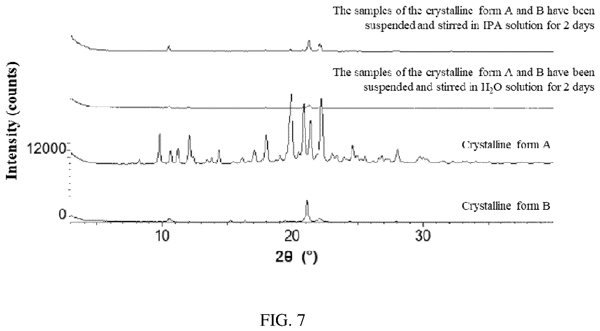

Study on the stability transformation relationship of the crystalline forms:
The transformation relationship between the crystalline forms was studied by a suspension competition experiment in the solution. The suspension competition between the crystalline form A and the crystalline form B was carried out at room temperature. The crystalline form A was obtained by adding equal amount of the crystalline form A/B to the saturated solution of isopropyl alcohol and water with suspending and stirring for 2 days. It shows that the crystalline form A has better thermodynamic stability at room temperature. The XRPD pattern of the suspension competition experiment between the crystalline forms is shown in FIG. 7.

Based on the above results of hygroscopicity and stability studies, it can be seen that the crystalline form A of the compound (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one of formula (I) has excellent stability and hygroscopicity, and more drug like.

The above examples are only intended to illustrate the technical concept and characteristics of the present invention, which is intended to enable persons familiar with the technology to understand the content in the present invention and implement it accordingly, and shall not limit the scope of protection of the present invention in this way. Any equivalent variation or modification made in accordance with the essence of the present invention shall fall within the scope of protection of the present invention.

## Claims

1. A crystalline form of compound (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one of formula (I), 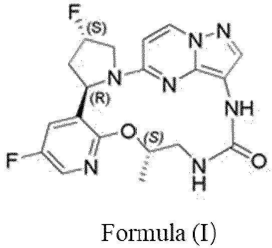 which is crystalline form A, wherein an X-ray powder diffraction pattern obtained from a CuKα source has characteristic peaks at 2θ values of 19.9°±0.2°, 20.9°±0.2°, 22.2°±0.2°.

2. The crystalline form A according to claim 1, wherein the X-ray powder diffraction pattern of crystalline form A further has characteristic peaks at one or more of the 2θ values of 21.4°±0.2°, 9.8°±0.2°, 12.1°±0.2°.

3. The crystalline form A according to claim 2, wherein the X-ray powder diffraction pattern of crystalline form A further has characteristic peaks at one or more of the 2θ values of 18.0°±0.2°, 24.6°±0.2°, 11.2°±0.2°.

4. The crystalline form A according to any one of claims 1-3, wherein the X-ray powder diffraction pattern of crystalline form A substantially as shown in FIG. 1.

5. A method for preparing the crystalline form A according to any one of claims 1-4, comprising the following steps:
the free base form of (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one was dissolved in a solvent in which the free base has a solubility greater than 2 mg/mL at room temperature or at 50 °C to obtain a clear solution, followed by cooling and standing to obtain the crystalline form A, or by evaporation to precipitate crystals to obtain the crystalline form A.

6. The method according to claim 5, wherein the solvent and mixed solvent are selected from methanol, acetone, dichloromethane, dimethyl sulfoxide or any mixture thereof.

7. A method for preparing the crystalline form A according to any one of claims 1-4, comprising the following steps:
dissolving the free base form of (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one in a solvent to obtain a clear solution, then adding an anti-solvent to crystallize; wherein the solvent is a solvent in which the solubility of (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one is greater than 2 mg/mL; the anti-solvent is a solvent in which the solubility of (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one is less than 2 mg/mL.

8. The method according to claim 7, wherein the solvent is selected from methanol, acetone, dichloromethane, dimethyl sulfoxide or any mixture thereof; the anti-solvent is selected from isopropyl alcohol, n-heptane, water or any mixture thereof.

9. The method according to claim 5 or 7, wherein the ratio of the solvent to (2²*R*,2⁴*S*,5*S*)-2⁴,3⁵-difluoro-5-methyl-4-oxa-7,9-diaza-1(5,3)-pyrazolo[1,5-a]pyrimidin -3(3,2)-pyridin-2(1,2)-pyrrolidincyclononan-8-one is 0.2-10 ml: 25 mg, preferably 0.8 ml: 25 mg.
